# EUROPEAN PATENT SPECIFICATION

(11) **EP 2 804 641 B1**
(45) Date of publication and mention of the grant of the patent: **30.03.2016**
(21) Application number: 13710060.8
(22) Date of filing: 15.01.2013
(51) Int. Cl.: A61M 1/36, A61M 39/16, A61M 39/22

(54) **EXTERNAL END DEVICE FOR PERMANENT CATHETERS SUITABLE FOR ISOLATING A LIQUID FLOW**
EXTERNE ENDVORRICHTUNG FÜR EINEN DAUERHAFTEN KATHETER ZUR ISOLIERUNG EINER FLÜSSIGKEITSSTRÖMUNG
DISPOSITIF D'EXTRÉMITÉ EXTERNE POUR CATHÉTERS PERMANENTS APPROPRIÉS POUR ISOLER UN ÉCOULEMENT DE LIQUIDE

(30) Priority: 16.01.2012 IT RM20120013
(43) Date of publication of application: 26.11.2014
(73) Proprietor: Grandolfo, Nicola, 70019 Triggiano (BA) (IT)
(72) Inventor: Grandolfo, Nicola, 70019 Triggiano (BA) (IT)
(74) Representative: Cardelli, Guido
(86) International application number: PCT/IT2013/000011
(87) International publication number: WO 2013/108280

(56) References cited:
- EP-A1- 0 070 087
- WO-A2-2005/046439
- WO-A2-2010/146614
- US-A- 3 827 439
- US-A- 5 324 274

## Description

### Technical field

The present invention relates to an external end device for permanent catheters suitable for isolating a flow of blood or other liquid from the environment, that is useful for example in hemodialysis, peritoneal dialysis and chemotherapy.

### Background art

The Patent Application No. PCT/IT2010/000269 of the same Applicant discloses an external end device for permanent catheters, comprising a container that can be connected on one side to two catheters, and on the other side to a closure lid containing a disposable absorbent material impregnated by an antiseptic substance. Such a container houses two taps being provided with knobs operable from outside of the container. The two taps have, on one side, first connectors for the connection to the catheters and, on the other side, second connectors projecting from the container for the connection to an external equipment The first connectors are connected to the catheters that exit the container through at least a flexible supporting tube section, connected in turn to the container. The supporting tube section holds externally a cuff designed to be positioned in the subcutaneous tissue of the patient's body. The second connectors are provided with caps surrounded by the disposable absorbent material that is received in the closure lid. The closure lid makes the absorbent material adhere to the caps, and covers the caps for protecting them externally from a bacterial attack by means of the antiseptic substance by which the absorbent material is impregnated.

The knobs positioned outside the container operate the taps connected to the catheters. Each tap drives a ball inside a respective duct connected to the catheter, for opening and closing the blood flow when necessary. Thus the ball is a foreign object in contact with the blood, and there is a risk that what must be aseptic can be attacked by pathogens. Stresses on the blood that are caused by the contact with the ball and by the flow interruption should not be undertaken.

### Summary of the invention

The present invention aims at overcoming the above mentioned drawbacks and troubles.

An object of the invention is to provide an external end device for permanent catheters that optimises the insulation of the catheters as well as the opening and closing means thereof from the external environment, and then to prevent pathogen attacks.

Another object of the invention is to reduce friction stresses and shear stress, that the blood undergoes when passing through a valve, e.g. a ball valve, and when the blood flow is interrupted by the ball.

Further an object of the invention is to provide an external end device that allows the real closed position of its taps to be checked.

For achieving the above objects the invention provides an external end device for permanent catheters as further disclosed in claim 1 for isolating a flow of a liquid from the environment, the end device comprising a body that can be connected on one side to two catheters, and on the other side to either a closure lid if there is no flow or to connectors for a external treatment equipment when there is a flow, and two pinch valves for interrupting the liquid flow in the two catheters.

### Brief description of drawings

Further features and advantages will be more evident in the present description of a preferred and not exclusive embodiment of an external end device for permanent catheters for isolating a flow of a liquid from the environment shown by way of an example and not limiting way with the aid of the enclosed drawing sheets in which:
Figure 1 shows a general perspective view of a embodiment of the device according to the invention, in a closed position;
Figure 2 shows an exploded perspective view of the device in Figure 1;
Figure 3 shows a longitudinally cross-sectioned partially exploded perspective view of the device in Figure 1;
Figure 4 shows an exploded side view of the device in Figure 1; and
Figure 5 shows an enlarged view of components of the device in Figure 2.

### Detailed description of embodiment

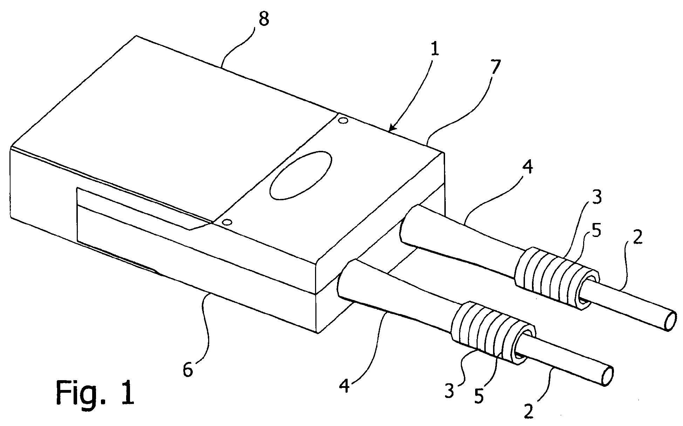

Referring to Figure 1 that is a general perspective view of a embodiment of the device according to the invention, in a closed position, i. e. when there is no liquid flow, a body of the device connected to two catheters 2, 2 is indicated as 1. Two subcutaneous cuffs are designated as 3, 3 that surround two supporting tube sections 4, 4 projecting from the device body 1 and connected thereto. According to another not shown embodiment the pair of catheters can be replaced by one dual lumen catheter, i.e. having two ways.

For convenience same reference numerals are used for indicating equal parts and in the following the description of the features of a part is implicitly valid also for the other identical part. Indicated as 5 in the subcutaneous cuffs is a silver wire emerging from the subcutaneous cuff 3 for a katadyn effect or purification by katadyn process, as known.

The body 1 comprises a base element 6 and a covering element 7. Indicated as 8 is a closure lid for the body 1. The body 1 in its side opposite to two catheters 2, 2 is closed, when there is no liquid flow, by means of the closure lid 8, as shown in Figure 1. Otherwise, when there is a liquid flow, the body 1 has connectors as seen in the following figures, for the connection to a not shown external treatment equipment.

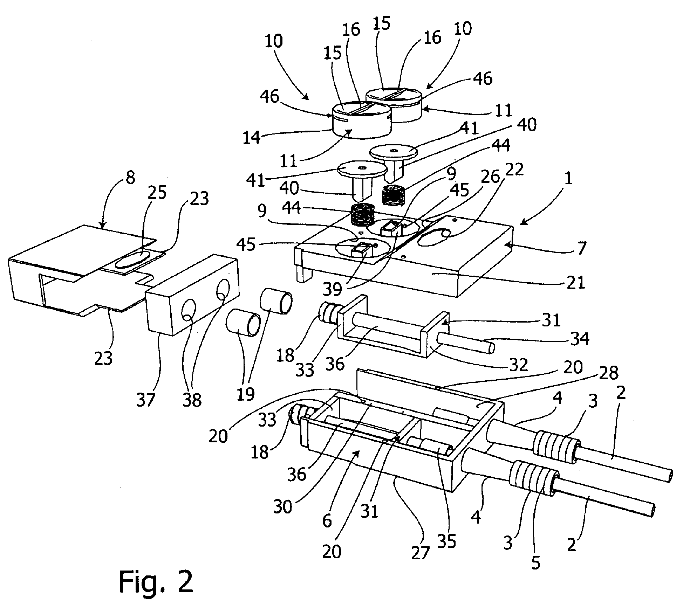

In Figure 2 that is an exploded perspective view of the device in Figure 1, the base element 6 is shown separated from the covering element 7 and the closure lid 8.

Formed in the covering element 7 are cylindrical seats 9, 9 and flow control units 10, 10, which are housed therein. The flow control units 10, 10 together with the elastic tubes, which are housed in the base element 6 and described in the following, form so-called pinch valves. The flow control units 10, 10 comprise knobs 11, 11 having a side wall 14 and a top 15. Formed on the top 15 are recesses for creating a diameter ridge 16 for an easy grip of the knobs 11, 11 for their rotation. The diameter ridges 16 indicate also the open-closed position respectively, of the pinch valves. As shown in the following figures a pin 17 is hanging from the top 15, inside each knob 11.

In their side facing an external treatment equipment not shown in the figures, as already said, the pinch valves have connectors 18, 18, for example Luer Lock, provided with caps generally indicated as 19. The covering element 7 is fixed to the base element 6, for example by screws in holes 20.

Formed in a raised portion 21 of the covering element 7 is an elliptical-shaped upper cut-out 22 to allow the closure lid 8 to be snap hooked. For this purpose, the closure lid 8 has a pair of plates 23, 23, singularly bearing a portion 25 projecting outside. When the closure lid 8 engages the body 1, the plates 23, 23 are inserted in through slots 26 in the raised portion 21 of the covering element 7, and in a lowered portion 27 of the base element 6, where the slot 26 is not shown in Figure 2.

The closure lid 8 serves to protect the knobs 18, 18 as well as the respective caps 19, when the end device according to the invention is dosed, in the case, for example, the end device is removed by the external treatment equipment and there is no liquid flow.

As will be seen hereinafter, in this condition the closure lid 8 covers and protects from inddental operations also the knobs 11, 11 in their lowered position. On the contrary, the closure lid 8 can not be inserted in the body 1 when the knobs 11, 11 are in their raised position, and this ensures the effective closed position of the pinch valves.

The base element 6 has a prismatic shape, although this form should not be construed as limiting. The interior of the base element 6, which is open at the top, is hollow, and may be divided longitudinally into two compartments 28, 28 by a separation wall 30 if any.

The compartments 28, 28 house two substantially U-shaped rigid cradles 31, respectively, each cradle having a first wall 32 facing the catheters 2, 2 and a second wall 33 opposite to the first wall 32. In any rigid cradle 31, the first wall 32 is joined to the second wall 33 by means of a base (not denoted with a reference numeral) which acts as an anvil for the pinch valves. The two rigid cradles 31 are removably anchored to the base element 6 through connecting means, for example, mortise and tenon, not shown in more detail in the figures.

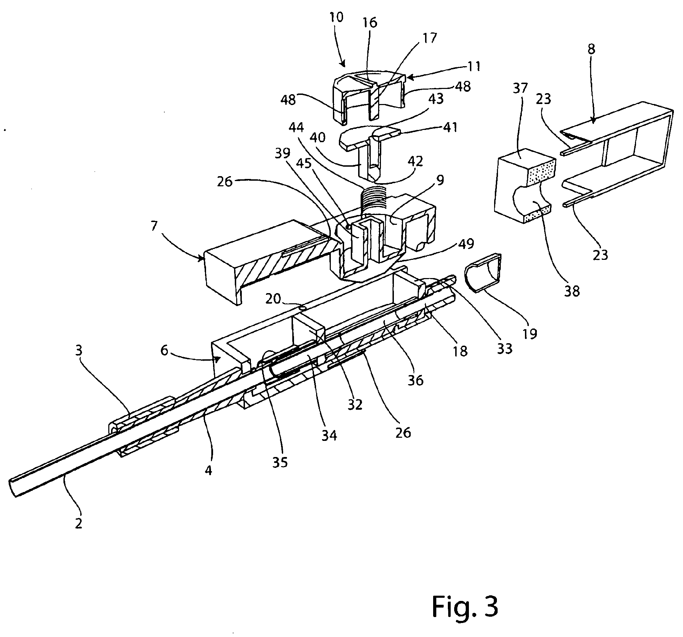

With reference to Figure 2 as well as to Figure 3, which is a partially exploded longitudinal cross-section of an isometric view of the body 1 according to the invention, the end device according to the invention is shown to be axial-symmetric, and the features discussed for a part thereof identically apply also for the other part. The first wall 32 of the rigid cradle 31 is crossed by a through fitting 34, on which the catheter 2 is press fitted. The connection between the through fitting 34 and the catheter 2 is ensured by a retaining means 35.

The second wall 33 of the rigid cradle 31 supports the connector 18 provided with a cap 19. Within the rigid cradle 31 there is an elastic tube 36 that connects the through fitting 34 with the corresponding connector 18. As previously mentioned, the elastic tube 36 together with the flow control unit 10 forms a pinch valve in the end device according to the present invention. Inside the closure lid 8 there is housed a prismatic shaped disposable absorbent material 37, which is impregnated with an antiseptic substance. The disposable absorbent material 37, preferably spongy, is inserted mainly on the caps 19 thanks to two corresponding spaces 38 made in the absorbent material 37. The diameter of the space 38 will be slightly lower than that of the caps 19, so that the absorbent material 37 can adhere on them by protecting them with the action of the antiseptic substance.

In Figure 3 there are shown with greater clarity the parts already described in the previous figures. In particular the cylindrical seat 9 and the flow control unit 10 with its knob 11 are shown in cross-section.

The cylindrical seat 9 is formed in the covering element 7 and is closed at its bottom. Centrally in the cylindrical seat 9 there is a hollow body 39 shaped, at least inside, as a rectangular cross-sectioned parallelepiped without bases, which has its pair of major faces arranged in vertical planes orthogonal to the direction of the elastic tube 36. The outer shape of the hollow body 39, which is not critical, is also parallelepiped in the shown embodiment.

The flow control unit 10 comprises a rectangular cross-section vertical pinching element 40, whose external dimensions correspond to those of the internal hollow body 39 In order to permit the vertical pinching element 40 to slide in the body 39, and a horizontal flat top 41 in a circular shape. The vertical pinching element 40 ends in its side opposite to the flat horizontal top 41 with a wedge-shaped lower end 42. The vertical pinching element 40 has a vertically elongated central cylindrical cavity 43, adapted to receive the pin 17 of the knob 11 hanging in the center towards the inside from its top 15 (Figure 3). The knob 11 engages the vertical pinch element 40 as an operation member for the movement of the latter.

A helical spring 44 is placed around the hollow body 39 in abutment between the bottom of the cylindrical seat 9 and the horizontal flat top 41 on its lower side.

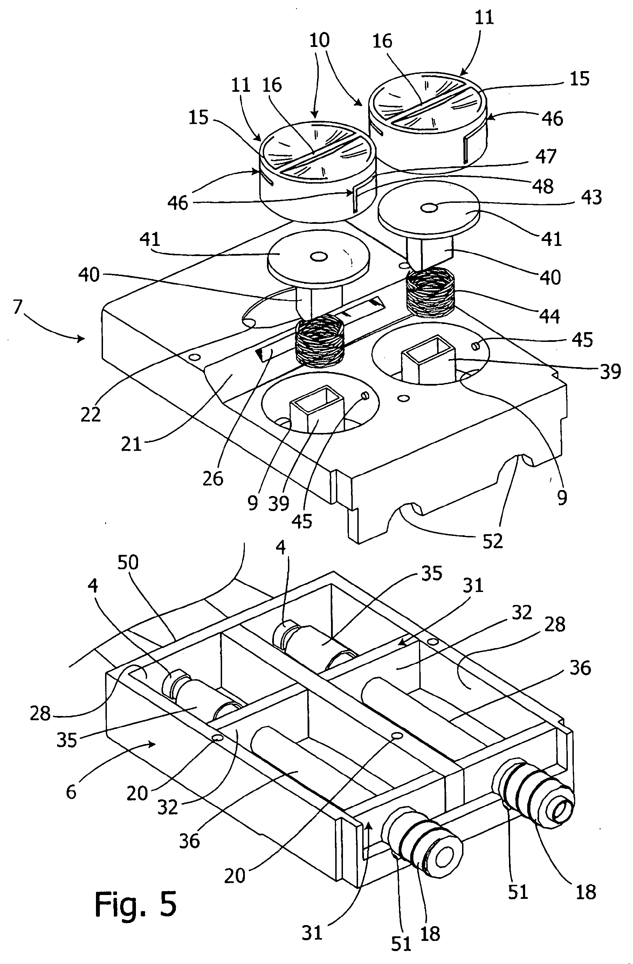

As shown in more detail in Figure 5, which is an enlarged view of Figure 2 but represents only the base element 6 and the covering element 7, placed in a suitable position on the lateral surface of the cylindrical seat 9 is a pair of pins 45. On the side wall 14 of each knob 11 there is a pair of L-shaped grooves 46, each of them being intended to receive a pin 45 projecting from the lateral surface of the cylindrical seat 9. Each L-shaped groove 46 has a horizontal section 47 of length equal to a quarter of the circumference of the knob 11 and a vertical section 48 facing downwards. The pin 45 constitutes a fixed cam, and the knob 11 equipped with the L-shaped groove 46 represents the cam follower. The vertical section 48 of an L-shaped groove 46 is located in the side wall 14 of the knob 11 and is diametrically opposite to that of the other L-shaped groove 46. The vertical section 48 is so high that, when the pin 45 is located in the lower end of the vertical section 48 of the L-shaped groove 46 in which it is received, the horizontal flat top 41 of the vertical pinching element 40, and consequently the knob 11, are pushed upward by the helical spring 44. In this position of the pinch valve, the wedge-shaped lower end 42 of the vertical pinching element 40 does not deform the elastic tube 36. When the knob 11 is manually pinched so that the pins 45, 45 are located in the respective opposite corners between the vertical section 48 and the horizontal section 47 of the L-shaped grooves 46, the wedge-shaped lower end 42 of the vertical pinching element 40 deforms the elastic tube 36 by closing the lumen thereof. The further rotation of the knob 11 so as to bring the pins 45, 45 in the horizontal section 47 of each the L-shaped groove 46 continues reliably the pinching of the elastic tube 36, while maintaining the interruption of the liquid flow internally. It was decided to use two pins 45, 45 and two L-shaped grooves 46, 46 for each knob 11 in order to retain always the same structure in its rotation and in its vertical translation.

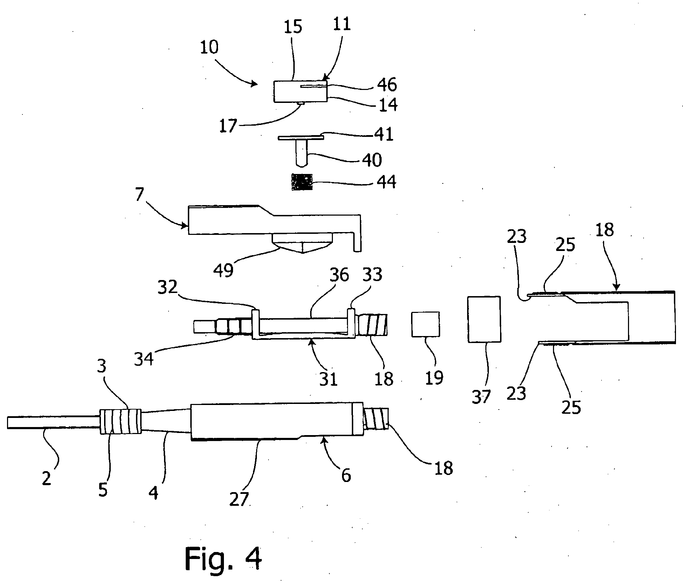

Advantageously, with reference to the Figure 3, below the cylindrical seat 9 opened at the bottom in the hollow body 39, a septum 49 of elastically deformable plastic material best seen in the Figure 4 is placed, which shows an exploded side view of the device in the Figure 1. The septum 49 is interposed as a mechanical protection between the elastic tube 36 and the wedge-shaped lower end 42 of the vertical pinching element 40. Furthermore, the septum 49 provides a full insulation of the internal environment of the base element 6, in which the elastic tube 36 is located, from the outside environment, when the covering element 7 is closed and secured on the base element 6.

When the body 1 is assembled, each vertical pinching element 40 of the flow control unit 10 is inserted in the corresponding hollow body 39 of the respective cylindrical seat 9 in the covering element 7 of the device, with the wedge-shaped lower end 42 being arranged perpendicular to the longitudinal direction of the elastic tubes 36. The rectangular shape of the vertical pinching elements 40, 40 and of the hollow bodies 39, 39 prevents the rotation of the corresponding flow control units 10, 10 around the vertical axis.

Each knob 11 is positioned by inserting its pivot 17, hanging down from its top 15, in the cylindrical cavity 43 of the corresponding vertical pinching element 40, while each pin 45 of the cylindrical seat 9 is steadily received in the L-shaped grooves 46 provided in the side wall 14 of the knob 11. The constraint represented by the engagement of the pin 45 in the L-shaped groove 46 gives the knob 11 freedom of rotating for a quarter of a circumference with respect to the cylindrical seat 9.

The relative positions of the diameter gripping ridge 16, of the L-shaped grooves 46 on the knob 11 and of the pins 45 on the cylindrical seat 9, are chosen so that, in the closed position of the pinch valve, the diameter gripping ridge 16 of the knob 11 assumes a position orthogonal to the longitudinal direction of the elastic tubes 36.

With reference to Figure 5 the catheters 2, 2 being surrounded by the supporting tube sections 4, 4 are shown passing through the base element 6 in its rear wall 50, and the connectors 18, 18 that are retained by the second wall 33 of the rigid cradle 31 frontally exit the base element 6 through semicircular openings 51 and 52, which are formed in the base member 6 and in the covering element 7, respectively. However, other constructive solutions, different from those shown, are possible.

In the assembly of the base element 6 of the body 1, each catheter 2 is inserted through the subcutaneous cuff 3 and the supporting tube section 4 to be press-fitted into the through fitting 34.

Within each rigid cradle 31, one end of the elastic tube 36 is connected to each through fitting 34, while its other end is connected to the corresponding connector 18.

In order to complete the assembly of the body 1, the covering element 7 is fixed on the base element 6 by means of screws or other.

In order to set the pinch valve in the closed position, the knob 11 is lowered and rotated clockwise. After the knob 11 is lowered, the pins 45 will be located in the corner between the vertical section 48 and the horizontal section 47 of each L-shaped groove 46 formed on the side wall 14 of the knob 11; by rotating ninety degrees clockwise the knob 11, each pin 45 moves, with respect to the knob 11, in the horizontal section 47 of its L-shaped groove 46. The downward displacement of the knob 11 is transmitted to the pinching element 40 which, by its wedge-shaped lower end 42, throttles the elastic tube 36 for preventing the passage of liquid.

The septum 49 matching the cylindrical seat 9 simultaneously serves to mechanically protect the elastic tube 36 from an excessive pinching stress, and to completely insulate the catheters from the outside environment with a result of a relevant reduction of risks of infection for the patient.

The simultaneous downward movement of the knob 11 and of the respective flow control unit 10 causes the compression of the helical spring 44.

During a reverse operation, after rotating the knob 11 counterclockwise and positioning each pin 45 in the vertical section 48 of each L-shaped groove 46, the helical spring 44 pushes up the flow control unit 10 together with the knob 11. In this way the elastic tube 36 is released from the previous pinching, and the liquid flow can resume.

After using the end device, the connectors 18, 18 are closed by the caps 19, 19, and the disposable absorbent material 37 impregnated with the antiseptic substance is inserted on the caps 19, 19 thanks to the two corresponding spaces 38. At the end the closure lid 8 is placed by inserting the plates 23, 23 thereof through the slots 26 formed in the raised portion 21 of the covering element 7 and, respectively, in the recessed portion 27 of the base element 6. When the full insertion of the plates 23, 23 in the body 1 is completed, the projecting portions 25 of the plates 23, 23 of the closure lid 8 snap into the slot 22 formed in the raised portion 21 of the covering element 7 and, respectively, in the lowered portion 27 of the base element 6. If it is not possible to put the closure lid on the body 1, this means that the complete closure of one or both of the pinch valves did not occur and that the user or the nurse or other person in charge must be concerned to execute it And this is a further element of safety for the patient.

## Claims

1. An external end device for permanent catheters that is suitable for isolating a liquid flow from the environment, the external end device comprising a body (1) connectable on one side to two catheters (2, 2), and on the other side to either a closure lid (8) or a treatment equipment, two valves being provided in the body (1) for interrupting the liquid flow in the two catheters, **characterised in that** said valves are so-called pinch valves, the body (1) comprising a base element (6) housing two elastic tubes (36, 36) acting as pinch valve sleeves that are compressible until the closure of their lumen and are situated downstream of their respective catheters (2, 2) and upstream of the respective connectors (18, 18), and a covering element (7) containing two flow control units (10, 10) for said pinch valves in such a position to interact with the two elastic tubes (36 , 36) in order to achieve their closure by pinching them and their opening by releasing them.

2. The device according to claim 1, **characterised in that** the base element (6) is divided into two compartments (28, 28) accommodating respective rigid cradles (31, 31), each rigid cradle (31) being adapted to support on one side a through fitting (34) for connecting each elastic tube (36) to the respective catheter (2) and on the other side a respective connector (18) connected to the same elastic tube (36).

3. The device according to claim 1, **characterised in that** each flow control unit (10) includes a vertical pinching element (40) engaged with the cover element (7), for its movement with respect to the elastic tubes (36, 36), a horizontal flat top (41) and a driving member for the movement of the vertical pinching element (40).

4. The device according to claim 3, **characterised in that** each driving member includes a knob (11) having a side wall (14) and a top (15) and being housed in the cover element (7) in a cylindrical seat (9) thereof that is provided with a partially closed bottom, said knob (11), in contact with the horizontal flat top (41), being adapted to be displaced downward against a counteracting spring (44) that is also housed in the cylindrical seat (9) and abutted against the horizontal flat top (41).

5. The device according to daim 4, **characterised in that** each vertical pinching element (40) is received in a prismatic guide in the form of a hollow body (39) open at both ends and contained within the cylindrical seat (9) so that the vertical pinching element (40) is adapted to pass through the bottom of the cylindrical seat (9).

6. The device according to daim 3, **characterised in that** a lower end (42) of each vertical pinching element (40) designed to come into contact with the corresponding elastic tube (36) is wedge-shaped.

7. The device according to daim 4, **characterised in that** applied to the covering element (7) underneath the cylindrical seats (9, 9), are two septa (49, 49) designed to interact with the vertical pinching elements (40, 40), said two septa (49, 49) being adapted to protect the elastic tubes (36, 36) and isolate the base element (6) from the environment

8. The device according to claim 4, **characterised in that** each flow control unit (10) includes a couple of diametrically opposite radial projections in the cylindrical seat (9), in the form of pins (45) acting as cams, and of L-shaped grooves (46) made on the side wall (14) of the knob (11) so to receive respective pins (45), the knob (11) acting as a cam follower.

9. The device according to daim 1, **characterised in that** said closure lid (8) includes protruding plates (23, 23) provided with projections (25, 25) adapted to engage slots in the body (1), so as to embrace the body (1) to cover the pinch valves and prevent the operation thereof.

## Patentansprüche

1. Externe Endvorrichtung für permanente Katheter, die geeignet ist, um einen Flüssigkeitsstrom von der Umgebung zu isolieren, wobei die externe Endvorrichtung einen Körper (1) umfasst, der an einer Seite an zwei Katheter (2, 2) angeschlossen werden kann, und an der anderen Seite entweder an einen Verschlussdeckel (8) oder ein Behandlungsgerät, wobei im Körper (1) zwei Ventile bereitgestellt sind, um den Flüssigkeitsstrom in den zwei Kathetern zu unterbrechen, **dadurch gekennzeichnet, dass** es sich bei den Ventilen um sog. Quetschventile handelt, wobei der Körper (1) ein Basiselement (6) umfasst, das zwei Schläuche (36, 36) enthält, die als Hüllen für die Quetschventile wirken und bis zum Schließen des Lumens zusammengepresst werden können und nach den jeweiligen Kathetern (2, 2) und vor den jeweiligen Konnektoren (18, 18) angeordnet sind, sowie ein Abdeckelement (7), das zwei Durchflussreglereinheiten (10, 10) für die Quetschventile enthält, und zwar in einer Position, in der diese mit den zwei Schläuchen (36, 36) interagieren, um deren Schließen durch Quetschen und deren Öffnen durch Freigabe zu erhalten.

2. Vorrichtung nach Anspruch 1, **dadurch gekennzeichnet, dass** das Basiselement (6) in zwei Bereiche (28, 28) unterteilt ist, die jeweilige steife Aufnahmevorrichtungen (31, 31) enthalten, wobei jede steife Aufnahmevorrichtung (31) geeignet ist, um auf einer Seite ein Durchführungsfitting (34) zu tragen, um jeden Schlauch (36) mit dem jeweiligen Katheter (2) zu verbinden, und auf der anderen Seite einen jeweiligen Konnektor (18), der mit demselben Schlauch (36) verbunden ist.

3. Vorrichtung nach Anspruch 1, **dadurch gekennzeichnet, dass** jede Durchflussreglereinheit (10) ein vertikales Quetschelement (40) beinhaltet, das mit dem Abdeckelement (7) in Eingriff ist, für dessen Bewegung zu den Schläuchen (36, 36), sowie ein waagerechtes flaches Oberteil (41) und ein Antriebsglied für die Bewegung des vertikalen Quetschelements (40).

4. Vorrichtung nach Anspruch 3, **dadurch gekennzeichnet, dass** jedes Antriebsglied einen Knauf (11) einschließt, aufweisend eine Seitenwand (14) und ein Oberteil (15) und untergebracht im Abdeckelement (7) in einem zylindrischen Sitz (9) davon, der mit einem teilweise abgeschlossenen Boden versehen ist, wobei der Knauf (11) in Kontakt mit dem waagerechten flachen Oberteil (41) ausgelegt ist, um nach unten gegen eine gegenwirkende Feder (44) verschoben zu werden, die ebenfalls im zylindrischen Sitz (9) untergebracht ist und gegen das waagerechte flache Oberteil (41) anschlägt.

5. Vorrichtung nach Anspruch 4, **dadurch gekennzeichnet, dass** jedes vertikale Quetschelement (40) in einer prismatischen Führung in Form eines Hohlkörpers (39), der an beiden Enden geöffnet und im zylindrischen Sitz (9) enthalten ist, aufgenommen wird, sodass das vertikale Quetschelement (40) ausgelegt ist, um durch den Boden des zylindrischen Sitzes (9) geführt zu werden.

6. Vorrichtung nach Anspruch 3, **dadurch gekennzeichnet, dass** ein unteres Ende (42) eines jeden vertikalen Quetschelements (40), das ausgestaltet ist, um in Kontakt mit dem entsprechenden Schlauch (36) zu kommen, keilförmig ist.

7. Vorrichtung nach Anspruch 4, **dadurch gekennzeichnet, dass** am Abdeckelement (7) unter den zylindrischen Sitzen (9, 9) zwei Scheidewände (49, 49) angebracht sind, die ausgestaltet sind, um mit den vertikalen Quetschelementen (40, 40) zu interagieren, wobei die zwei Scheidewände (49, 49) ausgelegt sind, um die Schläuche (36, 36) zu schützen und das Basiselement (6) von der Umgebung zu isolieren.

8. Vorrichtung nach Anspruch 4, **dadurch gekennzeichnet, dass** jede Durchflussreglereinheit (10) ein Paar diametral entgegengesetzte radiale Vorsprünge im zylindrischen Sitz (9) in Form von Zapfen (45), die als Nocken wirken, aufweist, sowie L-förmige Nuten (46), die an der Seitenwand (14) des Knaufs (11) ausgebildet sind, sodass jeweilige Zapfen (45) aufgenommen werden, wobei der Knauf (11) als Nockenstößel wirkt.

9. Vorrichtung nach Anspruch 1, **dadurch gekennzeichnet, dass** der Verschlussdeckel (8) hervorstehende Platten (23, 23) umfasst, die mit Vorsprüngen (25, 25) versehen sind, ausgelegt, um in Aussparungen im Körper (1) einzugreifen, sodass der Körper (1) umfasst wird, um die Quetschventile abzudecken und deren Betrieb zu vermeiden.

## Revendications

1. Un dispositif terminal externe pour les cathéters permanents qui permet l'isolation d'un flux de liquide à partir de l'environnement, le dispositif terminal externe comprenant un corps (1) pouvant être relié d'un côté à deux cathéters (2, 2) et, de l'autre côté, soit à un couvercle de fermeture (8) soit à un équipement de traitement, deux vannes étant prévues dans le corps (1) pour interrompre le flux du liquide dans les deux cathéters, **caractérisées en ce que** lesdites vannes sont ce que l'on qualifie de robinets-vannes à manchon déformable, le corps (1) comprenant un élément de base (6) contenant deux tubes élastiques (36, 36) faisant office de manchons de robinets-vannes qui sont compressibles jusqu'à la fermeture de leur canal et qui sont situés en aval de leurs cathéters respectifs (2, 2) et en amont des connecteurs respectifs (18, 18), et un élément de recouvrement (7) qui contient deux unités de contrôle du flux (10, 10) pour lesdits robinets-vannes à manchon, dans une position permettant d'interagir avec les deux tubes élastiques (36, 36) afin de procéder à la fermeture par pincement de ceux-ci et à l'ouverture par leur relâchement.

2. Dispositif selon la revendication 1, **caractérisé en ce que** l'élément de base (6) est divisé en deux compartiments (28, 28) accueillant des berceaux rigides respectifs (31, 31), chaque berceau rigide (31) étant adapté afin de supporter d'un côté un raccord passant (34) pour relier chaque tube élastique (36) au cathéter respectif (2) et de l'autre côté un connecteur respectif (18) relié à ce même tube élastique (36).

3. Dispositif selon la revendication 1, **caractérisé en ce que** chaque unité de contrôle du flux (10) comprend un élément de pincement vertical (40) en prise avec l'élément de recouvrement (7), pour son mouvement par rapport aux tubes élastiques (36, 36), un couvercle horizontal plat (41) et un élément d'entraînement pour le mouvement de l'élément de pincement vertical (40).

4. Dispositif selon la revendication 3, **caractérisé en ce que** chaque élément d'entraînement comprend un bouton (11) ayant une paroi latérale (14) et une partie supérieure (15) et étant logé dans l'élément de recouvrement (7) dans un siège cylindrique (9) qui est muni d'un fond partiellement fermé, ledit bouton (11), en contact avec le couvercle plat horizontal (41), étant adapté pour être déplacé vers le bas contre un ressort antagoniste (44) qui est également logé dans le siège cylindrique (9) et en butée contre le couvercle horizontal plat (41).

5. Dispositif selon la revendication 4, **caractérisé en ce que** chaque élément de pincement vertical (40) est reçu dans un guide prismatique sous la forme d'un corps creux (39) ouvert aux deux extrémités et contenu dans le siège cylindrique (9), de telle façon que l'élément de pincement vertical (40) soit en mesure de passer à travers le fond du siège cylindrique (9).

6. Dispositif selon la revendication 3, **caractérisé en ce qu'**une extrémité inférieure (42) de chaque élément de pincement vertical (40) conçue pour entrer en contact avec le tube élastique correspondant (36) est en forme de coin.

7. Dispositif selon la revendication 4, **caractérisé en ce que**, appliqués à l'élément de couverture (7) sous les sièges cylindriques (9, 9), se trouvent deux cloisons (49, 49) conçues pour interagir avec les éléments verticaux de pincement (40, 40), lesdites deux cloisons (49, 49) étant en mesure de protéger les tubes élastiques (36, 36) et d'isoler l'élément de base (6) de l'environnement.

8. Dispositif selon la revendication 4, **caractérisé en ce que** chaque unité de contrôle du flux (10) comprend un couple d'ergots radiaux diamétralement opposés dans le siège cylindrique (9), sous la forme de broches (45) faisant office de cames, et de rainures en forme de L (46) réalisées sur la paroi latérale (14) du bouton (11) de manière à accueillir les broches respectives (45), le bouton (11) agissant en tant que poussoir.

9. Dispositif selon la revendication 1, **caractérisé en ce que** ledit couvercle de fermeture (8) comprend des plaquettes en saillie (23, 23) équipées d'ergots (25, 25) adaptées pour engager les fentes dans le corps (1), afin d'englober le corps (1) pour couvrir les robinets-vannes à manchon amovible et empêcher le fonctionnement de ceux-ci.
